# EUROPEAN PATENT SPECIFICATION

(11) **EP 0 544 862 B1**
(45) Date of publication and mention of the grant of the patent: **18.03.1998**
(21) Application number: 92911960.0
(22) Date of filing: 29.05.1992
(51) Int. Cl.: G01N 33/92, G01N 33/68, G01N 33/564

(54) **Methods and compositions for the prevention, diagnosis, and treatment of inflammatory serosal diseases and related conditions**
4Verfahren und Zusammensetzungen zur Vorbeugung, Diagnose und Behandlung seröser Entzündungen und damit verbundener Störungen
Méthodes et compositions pour la prophylaxie, le diagnostic et le traitement des inflammations séreuses et des troubles associés

(30) Priority: 29.05.1991 US 705699
(43) Date of publication of application: 09.06.1993
(73) Proprietor: BAXTER INTERNATIONAL INC., Deerfield, IL 60015 (US)
(72) Inventor: DOBBIE, James, W., B-1400 Nivelles (BE)
(74) Representative: MacGregor, Gordon
(86) International application number: US9204432
(87) International publication number: WO9221981

(56) References cited:
- WO-A-89/01777
- ARCHIVES OF DERMATOLOGICAL RESEARCH vol. 283, no. 3, 1 March 1991, NEW YORK NY USA pages 141 - 148 M. ITO ET AL. 'Ultrastructural study of the skin in Sj gren-Larson syndrome.'
- THE BIOCHEMICAL JOURNAL, vol. 274, no. 1, 15 February 1991, London (UK), pp. 115 - 119 M.A. OOSTERLAKEN ET AL. 'Surfactant protein composition of lamellar bodies isolated from rat lung.'
- QUARTERLY JOURNAL OF MEDICINE, NEW SERIES vol. 72, no. 269, 1 September 1989, OXFORD UK pages 767 - 777 C.G. MACKWORTH-YOUNG ET AL. 'Antiphospholipid antibodies and disease.' cited in the application

## Description

### I. BACKGROUND OF THE INVENTION

The technical field of the invention relates to the prevention, diagnosis, and treatment of inflammatory and degenerative diseases of serous and related tissues (synovium, pleura, pericardium, peritoneum, lung, alimentary canal and other connective tissue) such as rheumatoid arthritis (RA), rheumatic fever, lupus erythematosus (SLE), psoriatic arthritis, spondyloarthropathies, Sjögren's syndrome, Reiter's syndrome, synovitis, osteoarthritis and tenosynovitis, and to pharmaceutical and/or biological compositions useful therefor. The etiology of such diseases is not completely understood but, in most cases, autoimmune mechanisms are thought to be principally involved. One other feature common to these diseases is that the serosal tissue or a related tissue is always affected. Serosal tissue consists of a mesothelial lining of cells and supporting fibro-connective tissue that covers body cavities including articular joints, pericardium, pleura, and peritoneum.

The following discussion presents background information on the etiology, clinical manifestations, autoimmunity, treatment, and diagnosis of inflammatory and degenerative diseases of serous and related tissues, as illustrated by the case of RA.

### A. ETIOLOGY OF RA

RA is a severe progressively crippling disease which affects about 1% of the world population, with about 20 million people suffering from a rheumatoid arthritic condition in the United States alone. The disease is 2 to 3 times more common in women, suggesting that sex hormones play a role in its development. Peak incidence is between 35 and 55 years in women and 40 and 60 years in men. The cause of RA is unknown and, although several environmental and infectious factors have been implicated in the fundamental pathologic process, their precise role has not yet been substantiated with regard to etiology. A genetic predisposition exists -- the presence of HLA-DR4 genetic loci has been correlated with an increased predisposition to RA.

### B. CLINICAL MANIFESTATIONS OF RA

The clinical manifestations of the disease are many fold. The most characteristic symptom of the disease is an inflammation of the joint-lining (synovial) membranes, their infiltration by white cells and an excessive production of the joint lubricating (synovial) fluid to cause the characteristic joint pain, swelling and deformities. Progression of the disease involves degradation and destruction of surrounding bone and cartilage. Although presenting predominantly as a joint disease, RA is a systemic disease which may explain the extra-articular manifestations of the disease in some patients. Among the non-joint manifestations is a dry eye Sjögren's syndrome condition - keratoconjunctivitis sicca - which is present in 15% of RA patients, especially in advanced stages. In addition, there can be pulmonary involvement in the form of diffuse fibrosis or pleural effusions. Evidence of previous pericarditis is often found on autopsy or by echocardiography but clinical manifestation of heart disease is rare. The clinical course of the disease is highly variable and unpredictable. Remissions and exacerbations are not uncommon, the former occurring in 10 to 20% of patients, usually in the earlier phases of the disease progression. However, 10% of patients suffer progressively crippling disease. Most patients respond to aggressive therapy but some 3% will ultimately be confined to bed. After 10 to 15 years, 50% of RA patients are still fully capable of carrying out normal activities but some 10% will have become nearly completely incapacitated.

### C. AUTOIMMUNITY IN RA

There is little doubt that autoimmunity plays a major role in RA, even if there is no evidence to support autoimmunity as the initial cause of the disease. One characteristic of the immunological involvement is the mononuclear cell infiltrates seen in the synovial membrane. This infiltrate contains T-lymphocytes, plasma cells and macrophages. T-cells are responsible for the release of cytokine factors involved as mediators in inflammation.

RA is frequently characterized by elevated serum and synovial fluid levels of rheumatoid factor (RF). Rheumatoid factors are auto-antibodies of the IgG, IgA and IgM isotopes specific for the Fc region of auto-antigenic IgG molecules responsible for triggering an autoimmune response in an as yet unknown way. It seems that the IgG molecules become altered in some way as the result of combination with an antigen and it is this complex which then serves as the immunogenic stimulus triggering the synthesis of RF auto-antibodies.

One explanation for the breakdown of tolerance to self-antigens is the "molecular mimicry theory". In this theory, induction of autoimmunity has been postulated to involve infection by agents such as viruses or bacteria, which may possess some antigenic determinants immunologically similar to the self-antigens. Circumstantial evidence in support of the molecular mimicry theory of RA induction is to be found in the observation that synovial fluid of some patients contains microbial antigens (i.e. bacterial lipopolysaccharide). Several studies have indicated a possibility of bacterial involvement such as salmonella, shigella, yersinia, mycoplasma, etc., suggesting that the original immune response to these organisms may have been involved in the cause of the disease. Nevertheless, it should be noted that the precise nature of a host target antigen attached as a result of an immune response to prior infections has not been identified. Lancet, 335, 685-688 (1990); Ann. Rheum. Dis., 40, 414-415 (1981) ; Current Opinion in Rheumatology, 1, 15 (1989).

### D. RA THERAPIES

A vast array of mainly pharmacological therapeutic approaches have been taken in the treatment of this disease. However, most of these approaches are associated with adverse side effects and are characterized by the feature that they are directed towards the treatment of the symptoms of the disease rather than their underlying immunological pathology.

The approach to therapy of RA employed for a particular patient depends upon the severity of the attack in that patient at the onset of the disease, and/or the state of its progression. With the disease state usually characterized by exacerbations and remissions, the treatment regimes must be changed and adapted accordingly to individual requirements. In general terms the drug therapeutic approach, as dictated by these highly variable factors in each patient, falls into three successive stages depending upon the successful sustained response or otherwise to a chosen treatment.

Those first employed are usually termed the front line drugs, aimed at minimizing and controlling the pain in RA: these include the simple analgesics, aspirin, and other non-steroidal anti-inflammatory drugs (NSAIDs). The latter to a lesser or greater extent are thought to exert some form of anti-inflammatory action which may or may not be responsible for any pain-deadening effect.

If, or when, the first line drugs prove only weakly effective or wane in their potency, recourse then has to be made to drugs aimed at altering the disease in some way and encouraging at least a sustained, if not permanent, remission. Such second line drugs are aimed more specifically at controlling and minimizing the more extreme forms of the inflammatory symptoms that develop with disease progression. The first line drugs are usually continued in use with the second line drugs, providing no adverse drug interactions occur. These second line drugs (or remittive agents) include gold compounds, penicillamine and certain antimalarial drugs.

Should second line drugs fail in certain patients then recourse is next made to immunosuppressive (cytotoxic) third line drugs, initially developed for cancer therapy. With autoimmune factors an essential feature in RA, the argument is that drugs suppressing the immune response should be effective in this context. However, the risks of developing the severe related side effects associated with these drugs prompts caution.

Conventional treatment of autoimmune diseases other than RA may similarly involve steroid therapy, immunosuppressive agents and anti-inflammatory agents. As in the case of RA these treatments have the disadvantage of treating the symptoms of the disease rather than the fundamental causative pathology. These treatments are palliative and are not without adverse side effects.

Ideally, treatment should be aimed at eliminating only the abnormal autoimmune response while leaving intact the ability to respond to other antigens.

### E. DIAGNOSIS OF RA

As far as the diagnosis of RA is concerned, it is felt by many authorities that a reliable test permitting the unambiguous diagnosis of the disease in its early stages would permit early therapeutic management prior to the development of irreversible lesions or deformities. RA diagnoses are currently made according to the American Rheumatism Association criteria. Among these 11 criteria are morning stiffness, pain on motion or tenderness in at least one joint, swelling of at least one joint, subcutaneous nodules, radiographic evidence of erosion, positive serologic test for rheumatoid factor, etc. RA diagnosis requires five of the classic RA criteria to be confirmed. None of these pathologic features are exclusively specific in the sense of being diagnostic for RA.

As far as in vitro testing is concerned, the presence of RF is the one indicator of the disease. RF is found in 80 to 90% of severe RA patients, but is usually only detectable some time after the initial disease has presented clinically. However, false positive reactions are not unusual. Examination of synovial fluid is routine in suspected RA cases, the objective being to assess the extent of the inflammation process by quantitating infiltrating cells which are mainly leukocytes, 85% of these being in the polymorphonuclear form. It can thus be seen that until now, no specific unique in vitro test is available allowing the early diagnosis of RA.

Further background information on RA may be found in: Scrip, Recent Trends in Research and Treatment in RA, PJB Publication (1988); New Eng. J. Med. 332, No. 18, 1277 (1990).

Biochem. J (1991) 274, 115-119, M.A. Oosterlaken et al "Surfactant Protein composition of lamellar bodies isolated from rat lung", discusses proteins obtained from lamellar bodies in the lung. WO-A-89/01777 discloses use of phospholipids for lubricating joints.

### F. OBJECTS OF THE INVENTION

The present invention addresses deficiencies in prior art relating to the diagnosis, treatment, and prevention of inflammatory and degenerative diseases of serous and related tissues, including synovium, pleura, pericardium, peritoneum, lung, alimentary canal, and connective tissue.

From one aspect, an object of the present invention is to permit diagnosis of inflammatory serosal disease and related conditions at an early stage, prior to the development of irreversible lesions or deformities.

A further object is to permit diagnosis of inflammatory serosal disease and related conditions accurately and reliably by simple in vitro testing.

From other aspects, further objects are to provide an effective treatment of inflammatory serosal disease and related conditions which are non-toxic and specific to an abnormal immune response that characterizes the disease, involve a restoration of bodily components depleted by the disease, and involve an interference with an autoimmune reaction characteristic of the disease.

A further object is to provide a vaccine that enables immunity to inflammatory serosal disease and related conditions.

A further object is to provide pharmaceutical and/or biological compositions for implementing the foregoing objectives.

Still further objects of the present invention will become apparent to one skilled in the art from the following description of the preferred embodiments.

### II. SUMMARY OF THE INVENTION

The present invention has several aspects, all involving inflammatory serosal disease and related conditions. The features of the invention are as disclosed in Claims 1, 5, 9, 10, 12 to 14, 20, 25, 30 and 33.

### III. DESCRIPTION OF THE PREFERRED EMBODIMENTS

The following describes methods and compositions for the prevention, diagnosis, and treatment of inflammatory and degenerative diseases of serous and related tissues, such as RA, rheumatic fever, SLE, psoriatic arthritis, spondyloarthropathies, Sjögren's syndrome, Reiter's syndrome, synovitis, osteoarthritis and tenosynovitis. The practice of the invention for the prevention, diagnosis, and treatment of RA is representative of such practice relating to the other enumerated diseases.

### A. LAMELLAR BODY DESCRIPTION

In accordance with the invention, it has been found that tissues affected by RA are predominantly those that contain cytoplasmic organelles called lamellar bodies. Lamellar bodies are cytoplasmic organelles of 1.5µ in diameter with a "finger print" pattern. Their lamellar structure is due to the presence of repeating electron-opaque and electronlucent zones. Lamellar bodies contain phospholipids and associated proteins. The phospholipids include phosphatidylcholine, phosphatidylinositol, phosphatidylethanolamine, sphingomyelin, lecithin, phosphatidylglycerol, phosphatidylserine, and lysolecithin. At least four major apoproteins, SP-A, SP-B, SP-C, and SP-D appear to be present in these organelles (recent tests have specifically demonstrated the presence of apoprotein SP-A in lamellar bodies of lung, synovial and mesothelial tissues), but, although these proteins are present in significant amounts, their possible role in organizing the structure of this organelle has not been fully addressed. These apoproteins have a major role in the transformation of the multilayered membranes of lamellar bodies into the complex three-dimensional lattice of the tubular myelin structure that develops from secreted surfactant.

Initiation of antigenic reactions against apoproteins but also to phospholipids such as diphosphatidylglycerol (cardiolipin) starts the immune process that leads to autoimmune diseases such as RA. Positive concentrations of antibodies to cardiolipin have previously been reported in patients with RA, SLE, and in other connective tissue diseases. In these autoimmune diseases, anti-phospholipid antibodies may persist for long periods of time and may even precede the onset of clinical symptoms by several years. Mackworth-Young, C.G., et al., "Anti-phospholipid antibodies and disease," Q. J. Med., 18, 767-77 (1989); Harris, E.N., "Anti-phospholipid antibodies," Clinics in Rheumatic Diseases, 11, No. 3, 591-609 (1985).

### B. ROLE AND LOCALIZATION

Until the early 1970s, distinctively structured cytoplasmic inclusions had not been recognized in type II pneumocytes because their predominantly lipid contents are readily extracted by the organic solvents used in the conventional processing of tissue for electron microscopy. Stratton, C.J., J. Ultrastruct. Res., 52, 309-322 (1982). Following the discovery that these inclusions are preserved by adding tannic acid to glutaraldehyde in the primary fixation of tissue, the role of the so-called lamellar bodies was rapidly elucidated.

The role of lamellar bodies in the storage and secretion of pulmonary surfactant is to lower surface tension -- by spreading as a monolayer, the surfactant lipids lower the contractile force of the air-liquid interface, reducing both the tendency of alveolae to collapse at end expiration and the transudation of fluids into the air spaces. Surfactant deficiency causes a disturbance of alveolar gas exchange, as illustrated by the clinical symptoms and signs of the neonatal respiratory distress syndrome (RDS), a leading cause of neonatal death. Farrell, P.M., and Avery, M.E., Am. Rev. Resp. Dis., 111, 657-668 (1975). Pleural fluid contains a mixture of phospholipids in which phosphatidylcholine is predominant.

A recent study in which these fixation techniques were applied to peritoneal tissue reveals the existence of lamellar bodies in parietal and visceral mesothelium in humans, monkeys, rabbits and mice. Dobbie, J.W. and Lloyd, J.K., Perit. Dial. Int., 9, 215-219 (1989); Dobbie, J.W., Am. J. Kid. Dis., XV, No. 2, 97-109 (1990). Arranged in concentric or parallel planar series, the lamellar structure and periodicity was found to be the same as that encountered in the type II pneumocytes in the same species processed for microcopy by the same technique.

Lamellar profiles were also identified in the endoplasmic reticulum and the golgi complex of mesothelial cells as first described in type II pneumocytes. In random sections of mesothelium, the number of lamellar bodies per average cross-section indicates a lower density per unit area than is found in type II pneumocytes.

These findings have also provided compelling evidence that a process of specialized biosynthesis and secretion of phospholipids similar to that established for type II pneumocytes also occurs in mesothelial cells.

It has been suggested that the ideal lubricant properties of phosphatidylcholine were derived from the attachment to the cell surface of the strongly positive choline end of the molecule, while the long hydrophobic tail of fatty acids presented a lubricant surface to the cavity. Hills, B.A., Butler, B.D., and Barrow, R.E., J. Appl. Physiol., 53, 463-469 (1982). Following the demonstration that peritoneal mesothelium synthesized phosphatidylcholine, it is suggested that sessile phosphatidylcholine molecules attached to sessile mesothelial microvilli is the principal arrangement for reducing serosal friction, not only in the peritoneum but in all serosal cavities. Dobbie, J.W., Pavlina, T., Lloyd, J., et al., Am. J. Kid. Dis., 12, 31-36 (1988). Lamellar bodies have also been demonstrated to be present in the cavities of the peritoneum, pericardium, pleura and colon, and in platelets.

### C. PRESENCE IN SYNOVIOCYTES

It has now been found that lamellar bodies are present in synovial cells in man and experimental animals. These lamellar bodies exhibit ultrastructural characteristics similar or identical to those found in mesothelial cells in other body cavities and type II pneumocytes in the lung. Suitable techniques for the isolation and culture of synoviocytes and the detection and localization of synovial lamellar bodies are illustrated in the following examples.

### EXAMPLE 1

### Isolation and culture of synoviocytes

Synoviocytes are obtained from a synovial sample by synovectomy from a non-rheumatoid patient. Synovium is dissected grossly and cut into very small pieces in 20 ml of Dulbecco's modified Eagle medium (DME), serum-free, containing 1 mg/ml collagenase type II (Sigma) and penicillin (5000 U/ml) and streptomycin (5 mg/ml). After 4 hours incubation at 37°C, the suspension is centrifuged for 10 minutes at 1400 rpm. The resultant cell pellet is resuspended in growth medium composed of DME medium supplemented with 20% fetal calf serum (FCS).

Cells are transferred to and grown in tissue culture plates at 37°C in a 5% CO₂ atmosphere. Culture dishes are maintained in the incubator and the growth medium is changed every two weeks. When cellular confluence is attained, a passage is carried out. Cells are then plated in multiwell culture dishes containing glass coverslips.

### EXAMPLE 2

### Detection of lamellar bodies by electron microscopy

Sub-confluent cultures of synovial cells as prepared in accordance with Example 1 are carefully resuspended and centrifuged for 10 minutes at 1400 rpm. The supernatant is then discarded and the cell pellet fixed in Sorensen's buffer containing 2% glutaraldehyde and 1% tannic acid for 24 hours at 4°C. After dehydration in acetone, the pellet is embedded in LR white resin. 50 mm ultrathin sections are cut and mounted on nickel grids for immunogold staining.

After incubation for 15 minutes in normal goat serum, the grids are incubated with a monoclonal antibody specific for lamellar body apoprotein diluted in Tris saline for 2 hours. After 15 minutes washing in Tris saline containing 0.2% BSA, a goat anti-mouse antiserum conjugated to 15 nm colloidal gold particles is applied for 1 hour. After several washes in Tris saline containing 0.2% BSA and in distilled water, immunological sections are stained for 5 minutes with 2% aqueous uranyl acetate. Gold particles are then quantified by electron microscopy allowing the detection of lamellar body apoprotein.

Detection of lamellar bodies by electron microscopy can also be performed using human synovial biopsies fixed and processed in the same way as synoviocyte cultures.

### EXAMPLE 3

### Immunohistochemical localization of lamellar bodies

Surgically resected synovial membranes are fixed in 15% formalin and embedded in paraffin. Sections 3 microns thick are used. The sections are deparaffinized with xylene and soaked in absolute methanol containing 0.3% hydrogen peroxide for 30 minutes at room temperature in order to eliminate endogenous peroxidase activity.

After incubation of the sections with 10% normal goat serum for 30 minutes at room temperature, monoclonal antibodies to surfactant apoproteins are added and the sections incubated overnight at 4°C. The sections are washed with Tris-buffered saline (pH 7.4), further incubated with biotinylated horse anti-mouse IgG (Vector Laboratories, Burlingame, California) for 30 minutes at room temperature, then treated with avidin-biotin peroxidase complex (Vector Laboratories) for 30 minutes at room temperature.

Peroxidase activity is visualized by incubating the sections in 0.05M ammonium acetate citric acid buffer containing 3.3'-diaminobenzidine hydrochloride (20 mg/ml) and 0.001% hydrogen peroxide and sections are counterstained with Mayer's hematoxylin.

As controls, normal mouse serum is used instead of first monoclonal antibody.

Synoviocytes are positively stained for surfactant apoprotein in the cytoplasm.

As illustrated in the foregoing examples, an organelle specific to tissues such as joints, lung, pleura, pericardium and peritoneum, commonly affected by RA and which offers itself as the initial target for an autoimmune process, whereby antibodies against this structure are induced, has now been identified for the first time.

### D. DIAGNOSTIC AND THERAPEUTIC USE

### 1. Diagnosis of RA

By enabling the identification of the antigen which is the target for the autoimmune reaction, the present invention allows the very early diagnosis of RA at a stage where patients are unlikely to have symptoms developed enough for making the diagnosis by prior art methods. The presently disclosed in vitro diagnostic assay allows the detection of RA in its initial phase, when the autoimmune disease may be reversible through the use of immunotherapies, and thus provides a unique and powerful tool. The invention is simple and easy to use and can be performed using standard immunoassays, provided the purified antigen (lamellar body or its constituents) and specific antiserum are available.

### a. Preparation of antigen

Antigenic material is suitably prepared by a variety of techniques. Antigenic material may be prepared by purifying antigen of animal origin or by genetic engineering.

Purified antigen of animal origin is suitably derived from synovial cells, mesothelial cells, or type II pneumocytes. The purified antigen is preferably derived from serosa and related tissues, that is, tissues that normally become inflamed or degenerated when affected by serosal disease. In order to help ensure mimicry of a disease-related antigen, the purified antigen used in connection with a particular serosal disease is also preferably derived from lamellar bodies that are substantially identical to lamellar bodies normally found in a tissue inflamed or degenerated by that particular disease. Thus, for example, the purified antigen used in the case of RA is preferably derived from synovial lamellar bodies.

The purification of lamellar bodies or their constituents is readily obtained using different body fluids, such as amniotic fluid, lavage fluid obtained from a patient treated for alveolar proteinosis, synovial fluid or peritoneal fluid. Platelets and tissues such as synovial membrane or lung tissues can also be considered as a source of lamellar bodies. Several species are suitable for this purpose, including man, rat, rabbit, sheep, pig and dog. The example that follows illustrates these techniques using pulmonary washings from a patient with alveolar proteinosis.

### EXAMPLE 4

### Purification of lamellar body apoproteins

Lamellar body apoproteins are purified from the saline lavage fluid obtained from a patient treated for alveolar proteinosis. After centrifugation at 12,000 x g, the pellet is delipidated, resuspended in a buffer (0.02 M K₂HPO₄, pH 8.0) and purified using an Affigel blue column according to the manufacturer's instructions (Bio-Rad, San Mateo, CA). Affigel blue column chromatography removes albumin and other serum proteins. The purified apoprotein is then precipitated with ethanol at -20°C overnight and concentrated by centrifugation. Protein concentration is determined by the Lowry method.

Pure preparations of lamellar body apoproteins and phospholipids can also be achieved by genetic engineering. The DNA corresponding to the apoprotein amino acid sequences are introduced into the genome of a microorganism which is used to synthesize the proteins in vitro. The following example illustrates suitable genetic engineering techniques.

### EXAMPLE 5

### Recombinant apoprotein production

The amino acid composition of synovial apoprotein is analyzed by hydrolysis with HCl at 110°C for 24 hours. Analyses are performed on an amino acid analyzer (model 121-M, Beckman Instruments Inc., Fullerton, California). Vapor-phase protein sequencing is performed on an amino acid sequencer (470A, Applied Biosystems Inc., Foster City, California) with an on-line model 120A HPLC. RNA is isolated from a sample of synovial membrane obtained during surgical removal.

After synthesis of double stranded cDNA using standard techniques, as discussed in Nature, 289, 555-559 (1981) and "Cloning of double stranded DNA" in Genetic Engineering: Principles and Methods (Setlow, J.K., and Hallaender, A., editors), Plenum Publishing Corp, N.Y., N.Y., 1, 15-49 (1979), cDNA is inserted into a plasmid. Transfection of bacterial strain with this plasmid is then carried out. Apoprotein clones are isolated and characterized using appropriate DNA probes.

DNA corresponding to the apoprotein is inserted in a Chinese hamster ovary (CHO)-suitable vector and CHO cells are transfected. Clone(s) expressing the gene coding for human synovial apoprotein are isolated and grown in culture. Master seed bank and working seed bank are prepared before large-scale production of the synovial apoprotein.

References teaching suitable genetic engineering techniques include: Maniatis, T., et al., Molecular Cloning, by Cold Spring Harbor Laboratory (1982); and Solid Phase Biochemistry (Scouten, W.H., editor), published by J. Wiley & Sons, N.Y., N.Y., at p. 631 (1983).

### b. Preparation of antisera

In the practice of the invention, specific antisera are suitably produced against lamellar body constituents in a variety of ways. The antibody can be polyclonal or monoclonal.

### EXAMPLE 6

### Polyclonal antiserum

Polyclonal antiserum is suitably raised in appropriate animals (rabbits, goats) using the purified lamellar body preparation. For this purpose, approximately 1 mg of total protein is sonicated for 10 seconds with 1 ml of Freund's complete adjuvant. This emulsion is then injected subcutaneously into numerous sites along the backs of the animals. Injections are repeated later at 2, 4 and 6 weeks. The titers of antibody are checked and the animals are bled 8 days after the final injection. The animals are periodically boosted to maintain elevated levels of antibody.

The immune animal serum is absorbed with normal human serum to remove antibodies against serum proteins, such as albumin and IgG which are normally present in pulmonary lavage fluid. IgG fraction for this antiserum is obtained by chromatography on a DEAE cellulose column.

### EXAMPLE 7

### Monoclonal antibody

Monoclonal antibody is suitably produced by murine hybridomas. A detailed method for preparing monoclonal antibodies is described in Methods in Enzymology, vol. 121, Academic Press (1986).

### c. Diagnostic techniques

Once a purified preparation of antigen and suitable antiserum are available, diagnosis of RA is suitably carried out by standard, known immunoassays, namely enzyme-linked immunoabsorbent assay (ELISA) or radioimmunoassays, immunofluorescence methods on frozen tissue sections, Western blot analysis or electron microscopy. These techniques are described below.

**Immunofluorescence**. This test consists of incubating slices of frozen synovial membrane tissue, of animal or human origin, with the patient's serum. If auto-antibodies directed against lamellar bodies or one of their constituents are present in the serum, these antibodies will bind to the tissue. Their presence is then revealed by treating the tissue with a fluorescent anti-human immunoglobulin, the slide being examined under a fluorescent microscope.

**Electron microscopy.** Detection of the presence in the patient's serum of auto-antibodies against lamellar bodies can also be performed by electron microscopy using immunogold staining. The following example illustrates this technique.

### EXAMPLE 8

### Detection by electron microscopy

Synovial biopsies from normal patients are fixed in glutaraldehyde 2% tannic acid 1% in phosphate buffer pH 7.4 for one hour. Tissue blocks are cut for electron microscopy, fixed in 1% osmium for 30 minutes, then washed in phosphate buffer and dehydrated through graded ethanol solutions. After embedding in Spurr, blocks are polymerized for 72 hours at 60°C. Thin sections mounted on nickel grids are incubated with the patient's serum for 30 minutes at room temperature, then with a colloidal gold solution bound to anti-human immunoglobulin for 1 hour at room temperature. After washings, they are stained with aqueous uranyl acetate and lead citrate.

Synoviocytes isolated from the patient's synovium are suitably employed in an alternative embodiment instead of synovial tissue in the case of diagnosis by immunofluorescence or electron microscopy. Surgical synovium biopsy is digested in Dubbelco's minimum essential medium (DMEM) by animal collagenase. Dissociated cells are collected by centrifugation and cultured in cluster dishes at 37°C in 5% CO₂ until confluence. For electron microscopy, cells are resuspended in 2% glutaraldehyde in buffer containing 1% tannic acid for 24 hours at 4%. The pellet is post-fixed in 1% osmium tetroxide and embedded in Spurr. Sections are cut and stained with uranyl acetate lead citrate.

**ELISA - radioimmunoassay**. Polystyrene microtiter plates, coated with a pure preparation of lamellar body, are incubated overnight with a sample of the patient's synovial fluid. After several washings to remove extraneous material bound to the tissue, a further incubation is carried out with an enzyme conjugated rabbit anti-human IgG antiserum, then with a reaction mixture containing the substrate corresponding to the enzyme. Many enzymes can be used with the corresponding substrates. In the case of radioimmunoassay, the anti-human IgG is radioiodinated. The following example illustrates this technique in more detail.

### EXAMPLE 9

### Diagnostic ELISA tests

A diagnostic ELISA test is used to determine the presence of autoantibodies directed against lamellar bodies or their constituents in the synovial fluid of RA patients. IgG and/or IgM antibodies to cardiolipin are analyzed in patient's serum with a quantitative ELISA (Cheshire Diagnostics, Chester, England). International standards are used. 96-well microtiter plates (NUNC Immunoplate, Denmark) are coated with the antigen by incubating each well with 200 µl of coating buffer (0.1M Na carbonate, pH 9.6) that contains 100 ng of purified surfactant apoprotein. The plate is incubated overnight at 4°C to facilitate adherence of the surfactant apoprotein to the bottom of the well.

The next morning, the antigen-coated plates are washed two times with PBS Tween buffer (200 µl/well/wash). After the wash, 200 µl of the patient's synovial fluid samples, diluted 1:1 and 1:10 in PBS, are incubated for 2-4 hours in the antigen-coated plates at room temperature. The plates are washed two times with PBS Tween buffer (200 µl/well/wash) and the bound antibodies are detected by incubating the wells with a second antibody, goat or rabbit anti-human IgG, conjugated to horseradish peroxidase (Collaborative Research, Lexington, MA), diluted 1:500.

After 2-4 hours of incubation at room temperature, the plate is washed two times with PBS Tween® buffer. A total of 200 µl of substrate (0.005% O-diphenylamine-0.03% H₂O₂ in distilled water) are added to each well. After a 30-minute incubation at room temperature the reaction is stopped by adding 50 µl of H₂SO₄ (2M) to each well, then the absorbance in each well is measured at 492 nm using an ELISA reader (Titertek Multiskan, Helsinki, Finland). The antibody anti-lamellar body constituents concentration is evaluated from the dilution of synovial fluid in which a positive reaction is observed.

**Blot analysis**. Immunoblotting with nitrocellulose can also be used as a diagnostic procedure for detection of autoantibodies against lamellar bodies. The following example is illustrative.

### EXAMPLE 10

### Diagnosis by immunoblot

The patient's serum samples are submitted to polyacrylamide gel electrophoresis (PAGE) using Pharmacia-LKB equipment. After electrophoresis, the gel is equilibrated in transfer buffer (25 mM Tris, 192 mM glycine and 20% methanol, pH 8.3) and a nitrocellulose membrane equilibrated in the same buffer is applied to the gel. The gel and the nitrocellulose sheet are placed between filter paper and sponge pads (Scotch Brite®, 6 mm), put into a vertical electroblotting apparatus filled with precooled transfer buffer, and blotted for 2.5 hours at 36V.

After the transfer is completed, the unoccupied protein binding sites on the nitrocellulose membrane are blocked by equilibration for 30 minutes in blocking buffer (500 mM phosphate buffer, pH 7.4, containing 0.9% sodium chloride and 0.5% Tween® 20). The nitrocellulose membrane with blotted proteins is incubated overnight at room temperature with 300 µl purified apoprotein diluted with 1500 µl blocking buffer. The membrane is washed 3 times for 10 minutes with saline containing 0.5% Tween® 20, then incubated for 1-2 hours at room temperature in monoclonal antibody against the apoprotein-enzyme conjugate, appropriately diluted in blocking buffer. After 2 washes with saline containing 0.5% Tween® 20, then with saline alone, the membrane is incubated with substrate solution until the desired intensity of bands is obtained. The water washed immunoblot is dried and preserved as desired.

### 2. Replacement Therapy

One therapeutic approach provided by the invention is the replacement of lamellar body secretion in deficient, damaged or pathologic serous and related tissues (synovium, pleura, pericardium, peritoneum, lung, alimentary canal and other connective tissue), whereby RA and other articular disease manifestations are suppressed or eliminated. The secretion is preferably specific to the diseased tissue, for example, lubricant surfactant secreted by synovial lamellar bodies is administered to a patient suffering RA.

Replacement is achieved by injection or infusion of preformed, in-vitro-produced lamellar body lubricant into body cavities (articular joints, pericardium, pleura, and peritoneum).

Infusion into the cavities, continually or intermittently, of the lamellar body preparation is able to restore lubrication and protection of the tissue surfaces during periods of wounding, surgical trauma, inflammation or depressed natural secretion.

Lamellar body preparation is suitably obtained from animal or human origin or by synthetic means using a combination of apoproteins produced by genetic engineering and synthetic phospholipids.

### EXAMPLE 11

### Replacement therapy

Isolation of lamellar bodies from amniotic fluid is performed using discontinuous sucrose density gradients. See Hallman et al., Pediatrics, 71, No. 4 (1983). The preparation is administered from an external or subcutaneous reservoir and conducted to the cavity or tissue by a conduit of suitable form and dimensions to accommodate the anatomical structure of the tissue perfused.

The lamellar body preparation contains a range of 10 to 100 mg/ml of phospholipids and 1 to 100 mg of apoproteins in a physiologically balanced electrolytic solution containing sodium chloride, calcium and magnesium.

The rate of infusions or frequency of injections is adjusted to maintain a concentration range of total phospholipids in the cavity (10 to 100 mg/l) which is normal for the joint and which will compensate for loss to the surrounding tissues or systemic circulation. The treatment continues until the physician determines that sufficient resolution of the joint disorder is reached.

### 3. Enhancement of Secretion

The biosynthesis and release of lubricant surfactant by lamellar bodies is induced or enhanced by drug therapy responsible for increasing the size and/or number of lamellar bodies.

These increases are accomplished by applying prior art techniques developed in connection with lamellar bodies of type II pneumocytes.

A wide range of morphological studies has revealed that the size and number of lamellar bodies in type II pneumocytes is responsive to a variety of neural, hormonal, nutritional and other environmental stimuli. Goldenberg, B.E., Buckingham, S., and Sommers, S.C., Lab. Invest., 16, 693-705 (1967); Wang, N.S., Kotas, R.V., Avery, M.E., and Thwelbeck, W.M., J. Appl. Physiol., 30, 362-365 (1971); Redding, R.A., Doublas, W.H.J., and Stein, M., Science, 175, 994-996 (1972); Picken, J., Lurie, M., and Klienerman, J., Am. Rev. Resp. Dis., 110, 746-753 (1974); Gail, D.B., Hassaro, G.D., Massaro, D., J. Appl. Physiol., 42, 88-92 (1977).

Stimulants of lubricant surfactant secretion include the β-adrenergic agonists (terbutaline), phosphodiesterase inhibitors (isobutyl-methylanthine), cholera toxin, 8-bromo-cyclic adenosine monophosphate (AMP), and corticosteroids. Stimulation by TPA, β-adrenergic agonists, and calcium ionophase (A22187) is additive, which implies that all three agents act via independent processes. Cholinergic agonists, especially pilocarpine, have been reported to stimulate surfactant secretion in vivo. Oyarzun, M.J., and Clements, J.A., Am. Rev. Resp. Dis., 117, 879-891 (1978). The use of drugs able to increase or restore secretion of lubricant surfactant by lamellar bodies is a pharmaceutical and/or biological treatment for all kinds of diseases involving deficiency or damage of serous and related tissues. Intra-articular injections or oral therapy of corticosteroid preparations is widely accepted as a successful means of alleviating RA joint symptoms, as illustrated by Sterling, L.P., "Rheumatoid arthritis: Current concepts and managements, part 2," Am. Pharm. (U.S.), NS30, No. 9, 49-54 (Sept. 1990).

### 4. Immunotherapeutic Treatment of RA

Treatment of RA and other associated autoimmune diseases is possible causing little if any toxicity. The absence of a clear-cut target antigen has limited the studies performed to date to the investigation of non-specific immunosuppression. The use of the antigenic properties of the lamellar body enables the immunotherapeutic treatment of RA and of all immune derangements of tissues in which lamellar bodies are located.

### a. Injection of immune complexes

A specific suppression of the immune response triggered against lamellar bodies is achieved by intradermal injection of immune complexes of lamellar bodies or their constituents, including phospholipids and apoproteins, and a specific monoclonal or polyclonal antibody. This approach has been demonstrated in the treatment of allergy, desensitization being effected by the administration of the allergen complexed with a specific antibody, the antibody preferably having been raised in the patient. St. Remy, J., et al., U.S. Patent 4,740,371. Antibodies are present in a ratio such that essentially all binding sites of the antigen are blocked by the antibody and hence the antigen produces effectively no immunological reaction when administered to the patient. A molar ratio of from about 1:1 to about 1:100, antigen to antibody, is suitable in the practice of the invention. Preferably, the antigenic constituent is specific to the diseased tissue, for example, the immune complex administered to a patient suffering RA preferably contains synovial lamellar bodies or their constituents.

### EXAMPLE 12

### Immune complex administration

The isolation of lamellar bodies (antigen) is accomplished according to the above. An antibody thereto is then generated. Three sources of antibody are suitably used: (a) immunized animals, (b) individual blood donors and pooled plasma from multiple donors, and (c) the patient him or herself.

**Antibody purification**. 5 ml of serum from rabbit immunized with pure lamellar body preparation are precipitated with 18% Na₂SO₄ at 37°C for 4 hours. The precipitate is washed and resuspended in 2.5 ml phosphate buffered saline (PBS) containing 1M NaCl and filtered through a 0.45µm filter. The solution is applied onto a TSK HF-55 (Merck, Darmstadt) gel column, chromatographed at a rate of 250 ml/h and recovered in 10 ml fractions. The two main peaks represent IgM and IgG. The IgG fraction is concentrated by ultrafiltration through an XM-100 Amicon® membrane to a volume of 1 ml and dialyzed for 24 hours against PBS with several changes of the dialysis bath.

**Preparation of the immunoadsorbent**. Purified lamellar bodies are coupled with carbodiimide to carboxylated agarose (CH-sepharose® 4B, Pharmacia). For this purpose, lamellar body solution is incubated at pH 4.5 with 0.1M carbodiimide and carboxylated agarose for 24 hours at 21°C. The remaining reactive groups on the solid phase are inactivated by incubation with lM glycine for 3 hours at 21°C. After several washings with 0.1M acetate buffer, pH 4.0, and 0.1M carbonate buffer, pH 8.3, both containing 0.5M NaCl, the immunoadsorbent is poured into a column (10 x 2 cm) and the IgG fraction applied. Specific antibodies are recovered after appropriate washings by elution with 50 mM citric acid. Eluted fractions are neutralized with drop-wise addition of 2M Tris-HCl buffer, concentrated on a YM 10 ultrafiltration membrane and dialyzed against PBS for 24 hours.

**Preparation of antigen-antibody complexes**. Antigen and antibody are mixed in a weight ratio of 1:10 to 1:100 in 9 g/l NaCl containing 0.3% human serum albumin and 4% phenol. All solutions are passed through a sterile 0.22µm filter and handled in sterile conditions. Enough antibody must be used so that there is no reaction to the antigen's antigenicity. The initial antigen dosage is preferably relatively small, a suitable range being from about 1 nanogram to about 100 micrograms. A suitable carrier is selected in accordance with prior teachings (see, e.g., U.S. Patent 4,740,371).

**Injections.** Intradermal injections on the internal side of the arm are repeated every week for 6 weeks, then every fortnight for a total of 3 months. In a typical scheme a volume of 100 µl containing 100 ng of antigen and 100 ng of antibody are used for each injection.

This treatment provides numerous advantages over conventional methods for the treatment of autoimmune diseases such as RA and SLE. The injection of this composition causes no toxicity and avoids the many other disadvantages of conventional treatments using drugs that suppress the immune response, which in turn suppresses the body's immunologic defense to antigens unrelated to the disease.

The specific mode of action by which the injection of immune complexes achieves its success is unknown but it is believed that the immune complexes stimulate the production of anti-idiotypic antibodies against the antibodies that are specific to the pathogenic antigen. The anti-idiotypic antibodies in turn suppress the production of antibodies at the cellular or possibly the humoral level. See U.S. Patent 4,740,371.

### b. T-cell vaccination

One other least toxic and suitable approach for specific immunological intervention in the disease process is one which has been termed T-cell vaccination (TCV). TCV is the therapeutic use of attenuated autoimmune T-cells as agents for inducing an immunological response against the pathogenic T-cells responsible for the destruction of target tissue. TCV in RA is based on the fact that T-cell reactivity against lamellar bodies is essential in the pathogenesis of the disease. These autoimmune T-cells are removed from arthritic patients (synovial fluid). Preparation of the vaccine entails in vitro expansion of T-cells derived from the patient and suitable treatments, such as UV irradiation or adjuration of cross-linking agents, processes which increase their potency. When they are injected back into the patient, the aggregated receptors evoke anti-idiotypic T-cells, thereby regulating the lymphocytes that cause the autoimmune disease.

Vaccination with T-cell lines that recognize critical self-antigens, attenuated so they can no longer cause disease, confer resistance to the specific autoimmune disease. It represents an alternative approach to exploit the ability of the immune system to respond to its own components.

A major obstacle to the use of T-cell vaccination for treatment of autoimmune disease is the need for antigen-specific T-cells to prepare efficient vaccines. Pure preparation of lamellar bodies provides the possibility of preparing a vaccine by specifically activating the appropriate T-cells.

### EXAMPLE 13

### Vaccination with T-cell lines

This example illustrates the use of TCV techniques in the treatment of inflammatory articular disease.

**Isolation of leukocytes**. 30 ml of peripheral blood are collected from each RA patient by venipuncture into heparinized tubes. Blood diluted 1:2 with sterile saline is layered carefully over Ficoll-Hypaque. No more than 2 times the volume of diluted blood has to be layered onto a given volume of Ficoll-Hypaque. The tubes are centrifuged for 30 minutes at room temperature at 400 x G. The mononuclear cells appear as a white band between the plasma and the Ficoll-Hypaque.

**Determination of optimal stimulus concentration**. This is done by [³H] thymidine pulsing allowing the determination of specific lymphocyte proliferation. The assay of the proliferative response of antigen selected lymphoblasts to the specific antigen is assayed in quadruplicate in flat bottom microtiter 96-well plates.

Each well contains 5 x 10⁴ specific lymphoblasts, 5 x 10⁴ autologous accessory cells in the form of irradiated cells and antigen in increasing concentrations (0-100 µg/ml) in 0.2 ml of proliferation medium. After 24 hours of incubation, the cultures are pulsed with [³H] thymidine for another 18 hours and then harvested using an LKB® cell harvester and the proliferative response is determined in a gamma counter.

Lymphocyte proliferation is expressed as cpm per cell number. Maximum cpm count allows the determination of optimal stimulus concentration.

**Culture conditions**. Cell suspension should be adjusted to 10⁶ cells per ml in medium containing 10% serum (human AB serum). Cultures are set up at three concentrations of cells (1 x 10⁶, 0.5 x 10⁶ and 1 x 10⁵) per well in the 24-well plates. Autologous irradiated mononuclear cells are added as feeder cells (5 x 10⁵ per well). Purified antigen preparation is added to each well at the concentration determined to be optimal in the system. The plates are transferred to a 37°C incubator gassed with 5% CO₂ and antigen-specific T-lymphoblasts are grown in propagation medium for 5-10 days, which is the time of maximum response to antigen. After a second antigenic stimulation, antigen-specific T-lymphoblasts are recovered.

**Vaccine preparation**. The potency of the vaccine is enhanced by aggregating the receptors of the T-lymphocytes into a mass. This is accomplished either physically (through hydrostatic pressure) or chemically (through agents that cross-link cell surface receptors such as formaldehyde). For pressure treatment, the T-lymphocytes are placed in 1.5 ml cold (4°C) phosphate-buffered saline (PBS) in an Eppendorf centrifuge tube. The tube is filled with fluid to avoid trapped bubbles and a short 21-gauge disposable syringe needle is pushed through the cap of the tube to equalize pressure. The tube is then introduced into a pressure cylinder (American Instrument, Silver Spring, Maryland) and filled with cold PBS. Pressure is applied slowly (over 4-5 minutes) to a level of 1250 bars for 15 minutes. Thereafter, the pressure is slowly released (over 4-5 minutes).

Cross-linking of membrane components is done by incubating 10⁸ T-lymphocytes in 5 ml of glutaraldehyde (0.3% in PBS) at room temperature for 15 minutes. The treated T-lymphocytes are washed six times by centrifugation in 50 ml PBS and used for vaccination (see Lider, et al., PNAS, 84, 4577-4580 (1987)).

The patient is inoculated subcutaneously in each arm with 0.5 ml of phosphate buffer saline containing 10 to 100 x 10⁶ autologous T-cells per ml and receives 3 similar booster inoculations at monthly intervals. Vaccines are suitably administered subcutaneously, intravenously or intraperitoneally since the effect of vaccination does not seem to be influenced by the administration route used.

T-cell vaccination can also be achieved using synthetic peptides corresponding to the lamellar body specific T-cell receptors.

### EXAMPLE 14

### Vaccination with T-cell receptors

After isolation of T-cell clones specifically activated by lamellar bodies or their constituents, as illustrated in Example 13, the genes which code for T-cell receptors are identified by appropriate c-DNA probes and sequenced. The amino acid sequence of proteins is deduced allowing the use of synthetic T-cell receptor peptides as vaccine suitable for administration.

Additional information relating to T-cell vaccination techniques is found in the prior art. Nature, 331, 171 (1988); Nature, 239, 181 (1988); Nature, 332, 843 (1988). Regarding T-cell receptors vaccination techniques, see PCT Application Publication No. WO9011294.

### c. Peptide competition for antigen presentation

In autoimmune diseases such as RA, the antigen is presented as a peptide on the surface of antigen-presenting cells. The association of this peptide with major histocompatibility complex (MHC) molecules is recognized by the T-cell receptor, leading to T-cell clones activation and the initiation of a specific immune response against the antigen.

Peptides of different amino acid sequences may compete for presentation by the same MHC molecule to T-lymphocyte and this has been shown to occur both in vitro and in vivo. Werdelin, O., J. Immunology, 129, 1883 (1982); Adorini, L., Nature, 334, 623 (1988); Adorini, L., Immunology Today, 11, No. 1, 21 (1990). Therefore, blocking the antigen-presenting capacity of disease-associated MHC class II molecules by an analogous peptide, unable to be recognized by the T-cell receptor, can interfere with the disease.

### EXAMPLE 15

### Peptide competition

After production of the recombinant synovial apoprotein (see Example 5), a library consisting of overlapping peptides, of between 8 to 20 amino acids in length, is synthesized using standard solid-phase techniques. Stewart, J.M., and Young, J.D., Solid Phase Peptide Synthesis, Pierce Chemical Co., Rockford, Illinois (1984).

Stimulatory peptides, responsible for the T-cell activation, are then identified by their ability to stimulate T-cell clones isolated from an RA patient. T-cell responses are determined in a standard lymphocyte proliferation assay as described in Example 13.

Analogous peptides to the stimulatory peptides are then produced by amino acid substitution. References teaching suitable amino acid substitution techniques include: Sambrook, Freitsch and Maniatis, Molecular Cloning (laboratory manual), Cold Spring Harbor Laboratory Press (1989).

The ability of these peptides to bind to MHC molecules without including any T-cell stimulation is then tested in vitro. The assay is based on the proliferation response of T-cell clones to the original stimulatory peptide, inhibited specifically by analogous peptides which compete for the MHC molecules on the antigen-presenting cells (see Example 13).

The blocking analogous peptide is administered to the patient at a dose of 10 to 1,000 micrograms in phosphate buffer saline. The correct dosage is determined by the inhibition assay. Vaccines are suitably administered subcutaneously once a week for a period of time determined by improvement of the patient's status and by the results of laboratory analysis.

### d. Antigen-specific tolerance induction

The identification of the specific triggering antigen in RA, i.e., lamellar body or its components, leads to a therapeutic approach such as the administration of the triggering antigen in a tolerogenic form. It has been observed that tolerance can be induced with high doses of antigen (referred to as high dose tolerance). This tolerization has been successfully used to eliminate ongoing autoimmune reactions to coagulation factors in hemophiliacs. Antibodies to Factor VIII arise in about 10% of patients with severe hemophilia A. It is possible to suppress antibodies to Factor VIII by giving prolonged infusions of very large quantities of Factor VIII over a period of one to two years. Prog. Clin. Biol. Res., 150, 181 (1984). Tolerance to Factor VIII has also been induced in hemophiliacs with inhibitors by administering massive doses of Factor VIII in combination with cyclophosphamide and intravenous IgG. New Eng. J. Med., 318, No. 15, 947 (1988).

Antigen-specific tolerance induction is employed against inflammatory articular disease such as RA, and other inflammatory and degenerative diseases of serous and related tissues. In a preferred embodiment, the antigen is recombinant apoprotein genetically reproduced, and the pharmaceutical composition is administered in accordance with prior art techniques that have been applied to enhance Factor VIII tolerance. Also the antigenic constituent is preferably specific to the diseased tissue, for example, the antigen of the pharmaceutical composition to be administered to a patient suffering RA is preferably synovial lamellar bodies or their constituents.

### EXAMPLE 16

### Tolerance induction

Cyclophosphamide is given from the first day of treatment, first intravenously, at daily doses of 12 to 15 mg per kilogram of body weight for 2 days, then orally, at daily doses of 2 to 3 mg per kg for 8 to 10 days.

The quantities of antigen, i.e., lamellar body or its components, used for injection can be equal or higher than 2.5 mg per kilogram for a daily dose and for a period of time determined by the improvement of the patient's status and by the results of laboratory analysis.

## Claims

1. A pharmaceutical composition for the treatment of a disease from a group of inflammatory and degenerative diseases of serous and related tissues comprising (a) an immune complex of an antigen selected from the group consisting of lamellar bodies, protein constituents of said lamellar bodies, and phospholipid constituents of said lamellar bodies, and an antibody specific to said antigen, and (b) a pharmaceutically acceptable carrier.

2. The composition of Claim 1, wherein said antigen and antibody are present in a ratio such that essentially all binding sites of said antigen are blocked by the antibody, whereby said antigen produces substantially no immunological reaction when administered to the patient.

3. The composition of Claim 1 or 2, wherein said lamellar bodies are substantially identical to lamellar bodies normally found in a tissue inflamed or degenerated by said disease.

4. A pharmaceutical composition for the treatment of inflammatory and degenerative diseases of serous and related tissues comprising purified lamellar bodies, protein constituents of said lamellar bodies, or phospholipid constituents of said lamellar bodies and a pharmaceutically acceptable salt.

5. A pharmaceutical composition comprising T-cells, or T-cell receptors, that recognise lamellar bodies from serous and related tissues, protein constituents of said lamellar bodies, or phospholipid constituents of said lamellar bodies, and a pharmaceutically acceptable carrier.

6. The composition of Claim 1, 2, or 5, wherein said lamellar bodies are lamellar bodies found in serosa and related tissues.

7. The composition of Claim 1, 2, 5 or 6, wherein said lamellar bodies are synovial lamellar bodies.

8. The composition of Claim, 1, 2, 5 or 6, wherein said lamellar bodies are substantially identical to lamellar bodies normally found in a tissue inflamed or degenerated by rheumatoid arthritis, rheumatic fever, lupus erythematosus, psoriatic arthritis, spondyloarthropathies, Sjögren's syndrome, Reiter's syndrome, synovitis, osteoarthritis, or tenosynovitis.

9. The composition of any preceding claim, wherein said protein constituents are selected from the group consisting of apoprotein SP-A, apoprotein SP-B, apoprotein SP-C, and apoprotein SP-D, and said phospholipid constituents are selected from the group consisting of phosphatidylcholine, phosphatidylinositol, phosphatidylethanolamine, sphingomyelin, lecithin, phosphatidylglycerol, diphosphatidylglycerol, phosphatidylserine, and lysolecithin.

10. Use in the manufacture of a pharmaceutical composition of an immune complex comprising an antigen selected from lamellar bodies, protein constituents of said lamellar bodies, or phospholipid constituents of said lamellar bodies, and an antibody specific to said antigen, for the purpose of treating an inflammatory or degenerative disease of serous and related tissues in a patient.

11. Use according to Claim 10, wherein said antigen and antibody are present in a ratio such that essentially all binding sites of said antigen are blocked by the antibody, whereby said antigen produces substantially no immunological reaction when administered to the patient.

12. Use in the manufacture of a pharmaceutical composition of β-adrenergic agonists (terbutaline), phosphodiesterase inhibitors (isobutylmethylanthine), cholera toxin, 8-bromo-cyclic adenosine monophosphate, TPA, calcium ionophase, cholinergic agonists, or corticosteroids, for the purpose of stimulating in a patient release of surfactant by lamellar bodies in serosa and related tissues inflamed or degenerated by disease.

13. Use, in the manufacture of a vaccine, of T-cell lines, or T-cell receptors that recognise lamellar bodies from serous and related tissues, protein constituents of said lamellar bodies, or phospholipid constituents of said lamellar bodies, for the purpose of vaccinating a patient against inflammatory and degenerative disease of serous and related tissues.

14. Use in the manufacture of a pharmaceutical composition of lamellar bodies, protein constituents of lamellar bodies, or phospholipid constituents of lamellar bodies for the purpose of vaccinating a patient in which such a disease is to be prevented, for the purpose of preventing inflammatory and degenerative diseases of serous and related tissues by inducing auto-immunological tolerance in a patient to lamellar bodies in said tissues, protein constituents of said lamellar bodies, or phospholipid constituents of said lamellar bodies.

15. Use according to Claim 11 or 14, wherein said lamellar bodies are lamellar bodies found in serosa and related tissues.

16. Use according to any one of Claims 11 or 14, wherein said lamellar bodies are substantially identical to lamellar bodies normally found in a tissue inflamed or degenerated by said disease.

17. Use according to any one of Claims 11 to 16, wherein said disease is rheumatoid arthritis.

18. Use according to any one of Claims 11 to 16, wherein said disease is rheumatic fever, lupus erythematosus, psoriatic arthritis, sponyloarthropathies, Sjögren's syndrome, Reiter's syndrome, synovitis, osteoarthritis, or tenosynovitis.

19. Use according to Claim 10, 11, 13 or 14, or any one of Claims 15 to 18 as appendant thereto, wherein said protein constituents are selected from the group consisting of apoprotein SP-A, apoprotein SP-B, apoprotein SP-C, and apoprotein SP-D, and said phospholipid constituents are selected from the group consisting of phosphatidylcholine, phosphatidylinositol, phosphatidylethanolamine, sphingomyelin, lecithin, phophatidylglycerol, diphosphatidylglycerol, phosphatidylserine, and lysolecithin.

20. A method of producing a pharmaceutical composition for treating inflammatory and degenerative disorders of serous and related tissues comprising the steps of identifying stimulatory peptides of lamellar body apoprotein origin that, when associated with major histocompatibility complex molecules, are recognised by T-cell receptors and initiate a specific immune response associated with such a disorder, producing a composition of analogous peptides capable of associating with said complex in competition with said stimulatory peptides but being unrecognizable by said T-cell receptors.

21. The method of Claim 20, wherein said apoprotein is from lamellar bodies found in serosa and related tissues.

22. The method of Claim 20, wherein said apoprotein is from lamellar bodies that are substantially identical to lamellar bodies normally found in a tissue inflamed or degenerated by said disorder.

23. The method of Claim 20, 21 or 22, wherein said disorder is rheumatoid arthritis.

24. The method of Claim 20, 21 or 22, wherein said disorder is rheumatic fever, lupus erythematosus, psoriatic arthritis, sponyloarthropathies, Sjögren's syndrome, Reiter's syndrome, synovitis, osteoarthritis, or tenosynovitis.

25. A method of manufacturing a pharmaceutical composition for the treatment of inflammatory and degenerative diseases of serous and related tissues comprising the step of admixing an antigen selected from the group consisting of lamellar bodies, protein constituents of said lamellar bodies, and phospholipid constituents of said lamellar bodies, and an antibody specific thereto in a ratio such that essentially all binding sites of said antigen are blocked by said antibody, whereby said antigen produces substantially no immunological reaction when administered to a patient suffering such a disease.

26. The method of Claim 25, wherein said lamellar bodies are lamellar bodies found in serosa and related tissues.

27. The method of Claim 25, wherein said lamellar bodies are substantially identical to lamellar bodies normally found in a tissue inflamed or degenerated by said disease.

28. The method of Claim 25, wherein said disease is rheumatoid arthritis and said lamellar bodies are synovial lamellar bodies.

29. The method of Claim 25, 26, 27 or 28, wherein said protein constituents are selected from the group consisting of apoprotein SP-A, apoprotein SP-B, apoprotein SP-C, and apoprotein SP-D, or said phospholipid constituents are selected from the group consisting of phosphatidylcholine, phosphatidylinositol, phosphatidylethanolamine, sphingomyelin, lecithin, phosphatidylglycerol, diphosphatidylglycerol, phosphatidylserine, and lysolecithin.

30. An *in vitro* method of diagnosing inflammatory and degenerative disease of serous and related tissues, comprising incubating an antigen selected from purified lamellar bodies, protein constituents of said lamellar bodies and phospholipid constituents of said lamellar bodies with a serous or related tissue sample, so that antibodies in the sample are bound to the antigen, and detecting the presence of the bound antibodies.

31. A method according to Claim 30, wherein the protein constituents are selected from apoprotein SP-A, apoprotein SP-B, apoprotein SP-C and apoprotein SP-D.

32. A method according to Claim 30 or 31, wherein said lamellar bodies are synovial lamellar bodies.

33. Device for use in diagnosing inflammatory and degenerative disease of serous or related tissues, comprising a microtiter plate having a well coated with an antigen selected from purified lamellar bodies, protein constituents thereof, or phospholipid constituents thereof.

34. Device according to Claim 33, wherein the protein constituents are selected from apoprotein SP-A, apoprotein SP-B, apoprotein SP-C and apoprotein SP-D.

35. Device according to Claim 33, wherein said lamellar bodies are synovial lamellar bodies.

## Revendications

1. Composition pharmaceutique pour le traitement d'une maladie de la famille des maladies inflammatoires et dégénératives de tissus séreux et apparentés, ladite composition comprenant (a) un complexe immun d'un antigène choisi parmi l'ensemble constitué par les corps lamellaires, les constituants protéiniques desdits corps lamellaires et les constituants phospholipidiques desdits corps lamellaires, et un anticorps spécifique dudit antigène, et (b) un véhicule pharmaceutiquement acceptable.

2. Composition suivant la revendication 1, dans laquelle lesdits antigène et anticorps sont présents selon un rapport tel que tous les sites de liaison dudit antigène sont essentiellement bloqués par l'anticorps, de façon que ledit antigène ne produise essentiellement aucune réaction immunologique quand il est administré à un patient.

3. Composition suivant la revendication 1 ou 2, dans laquelle lesdits corps lamellaires sont essentiellement identiques aux corps lamellaires se trouvant normalement dans un tissu enflammé ou dégénéré par ladite maladie.

4. Composition pharmaceutique pour le traitement de maladies inflammatoires et dégénératives de tissus séreux et apparentés, ladite composition comprenant des corps lamellaires purifiés, des constituants protéiniques purifiés desdits corps lamellaires ou des constituants phospholipidiques purifiés desdits corps lamellaires, et un sel pharmaceutiquement acceptable.

5. Composition pharmaceutique comprenant des cellules T ou des récepteurs de cellules T, qui reconnaissent les corps lamellaires de tissus séreux et apparentés, les constituants protéiniques desdits corps lamellaires ou les constituants phospholipidiques desdits corps lamellaires, et un véhicule pharmaceutiquement acceptable.

6. Composition suivant la revendication 1, 2 ou 5, dans laquelle lesdits corps lamellaires sont des corps lamellaires se trouvant dans la membrane séreuse et les tissus apparentés.

7. Composition suivant la revendication 1, 2, 5 ou 6, dans laquelle lesdits corps lamellaires sont des corps lamellaires synoviaux.

8. Composition suivant la revendication 1, 2, 5 ou 6, dans laquelle lesdits corps lamellaires sont essentiellement identiques aux corps lamellaires se trouvant normalement dans le tissu enflammé ou dégénéré par le rhumatisme articulaire, la fièvre rhumatismale, le lupus érythémateux, l'arthrite psoriasique, les spondylarthropathies, le syndrome de Sjögren, le syndrome de Reiter, la synovite, l'ostéoarthrite, ou la ténosynovite.

9. Composition suivant l'une quelconque des revendications précédentes, dans laquelle lesdits constituants protéiniques sont choisis parmi l'ensemble constitué par l'apoprotéine SP-A, l'apoprotéine SP-B, l'apoprotéine SP-C et l'apoprotéine SP-D, et lesdits constituants phospholipidiques sont choisis parmi l'ensemble constitué par les phosphatidylcholine, phosphatidylinositol, phosphatidyléthanolamine, sphingomyéline, lécithine, phosphatidylglycérol, diphosphatidylglycérol, phosphatidylsérine et lysolécithine.

10. Utilisation dans la préparation d'une composition pharmaceutique d'un complexe immun comprenant un antigène choisi parmi les corps lamellaires, les constituants protéiniques desdits corps lamellaires ou les constituants phospholipidiques desdits corps lamellaires, et un anticorps spécifique dudit antigène, dans le but de traiter une maladie inflammatoire ou dégénérative de tissus séreux et apparentés chez un patient.

11. Utilisation suivant la revendication 10, dans laquelle lesdits antigène et anticorps sont présents selon un rapport tel que tous les sites de liaison dudit antigène sont essentiellement bloqués par l'anticorps, de façon que ledit antigène ne produise essentiellement aucune réaction immunologique quand il est administré à un patient.

12. Utilisation dans la préparation d'une composition pharmaceutique d'agonistes β-adrénergiques (terbutaline), inhibiteurs de la phosphodiestérase (isobutyl-méthylanthine). toxine du choléra, monophosphate de 8-brome-adénosine cyclique, TPA, ionophase calcique, agonistes cholinergiques ou corticostéroïdes, dans le but de stimuler chez un patient la libération d'un surfactant par les corps lamellaires dans la membrane séreuse et les tissus apparentés enflammés ou dégénérés par la maladie.

13. Utilisation, dans la préparation d'un vaccin, de lignées de cellules T ou de récepteurs de cellules T qui reconnaissent les corps lamellaires de tissus séreux et apparentés, les constituants protéiniques desdits corps lamellaires ou les constituants phospholipidiques desdits corps lamellaires, dans le but de vacciner un patient vis-à-vis d'une maladie inflammatoire ou dégénérative des tissus séreux et apparentés.

14. Utilisation dans la préparation d'une composition pharmaceutique de corps lamellaires de tissus séreux et apparentés, de constituants protéiniques desdits corps lamellaires ou de constituants phospholipidiques desdits corps lamellaires dans le but de vacciner un patient devant être protégé d'une telle maladie, en vue de prévenir les maladies inflammatoires et dégénératives de tissus séreux et apparentés par induction chez ledit patient d'une tolérance auto-immunologique aux corps lamellaires dans lesdits tissus, aux constituants protéiniques desdits corps lamellaires ou aux constituants phospholipidiques desdits corps lamellaires.

15. Utilisation suivant la revendication 11 ou 14, dans laquelle lesdits corps lamellaires sont des corps lamellaires se trouvant dans la membranes séreuse et les tissus apparentés.

16. Utilisation suivant la revendication 11 ou 14, dans laquelle lesdits corps lamellaires sont essentiellement identiques aux corps lamellaires se trouvant normalement dans un tissu enflammé ou dégénéré par ladite maladie.

17. Utilisation suivant l'une quelconque des revendications 11 à 16, dans laquelle ladite maladie est le rhumatisme articulaire.

18. Utilisation suivant l'une quelconque des revendications 11 à 16, dans laquelle ladite maladie est la fièvre rhumatismale, le lupus érythémateux, l'arthrite psoriasique, les spondylarthropathies, le syndrome de Sjögren, le syndrome de Reiter, la synovite, l'ostéoarthrite, ou la ténosynovite.

19. Utilisation suivant la revendication 10, 11, 13 ou 14 ou l'une quelconque des revendications 15 à 18 qui en dépendent, dans laquelle lesdits constituants protéiniques sont choisis parmi l'ensemble constitué par l'apoprotéine SP-A, l'apoprotéine SP-B, l'apoprotéine SP-C et l'apoprotéine SP-D, et lesdits constituants phospholipidiques sont choisis parmi l'ensemble constitué par les phosphatidylcholine, phosphatidylinositol, phosphatidyléthanolamine, sphingomyéline, lécithine, phosphatidylglycérol, diphosphatidylglycérol, phosphatidylsérine et lysolécithine.

20. Procédé pour la préparation d'une composition pharmaceutique pour le traitement de désordres inflammatoires et dégénératifs de tissus séreux et apparentés, ledit procédé comprenant les étapes d'identification des peptides stimulants qui ont pour origine une apoprotéine de corps lamellaire, qui, quand ils sont associés avec des molécules d'un complexe principal d'histocompatibilité, sont reconnus par les récepteurs de cellules T et initient une réponse immune spécifique associée à un tel désordre, et de production d'une composition de peptides analogues capables de s'associer avec ledit complexe en compétition avec lesdits peptides stimulants mais non reconnaissables par lesdits récepteurs de cellules T.

21. Procédé suivant la revendication 20, dans lequel ladite apoprotéine est celle de corps lamellaires se trouvant dans la membrane séreuse et les tissus apparentés.

22. Procédé suivant la revendication 20, dans lequel ladite apoprotéine est celle de corps lamellaires qui sont essentiellement identiques aux corps lamellaires se trouvant normalement dans un tissu enflammé ou dégénéré par ledit désordre.

23. Procédé suivant la revendication 20, 21 ou 22, dans lequel ledit désordre est le rhumatisme articulaire.

24. Procédé suivant la revendication 20, 21 ou 22, dans lequel ledit désordre est la fièvre rhumatismale, le lupus érythémateux, l'arthrite psoriasique, les spondylarthropathies, le syndrome de Sjögren, le syndrome de Reiter, la synovite, l'ostéoarthrite, ou la ténosynovite.

25. Procédé de fabrication d'une composition pharmaceutique pour le traitement de maladies inflammatoires et dégénératives de tissus séreux et apparentés, ledit procédé comprenant l'étape de mélange d'un antigène choisi parmi l'ensemble constitué par les corps lamellaires, les constituants protéiniques desdits corps lamellaires et les constituants phospholipidiques desdits corps lamellaires, et un anticorps spécifique dudit antigène selon un rapport tel que tous les sites de liaison dudit antigène sont essentiellement bloqués par ledit anticorps, de façon que ledit antigène ne produise essentiellement aucune réaction immunologique quand il est administré à un patient souffrant d'une telle maladie.

26. Procédé suivant la revendication 25, dans lequel lesdits corps lamellaires sont des corps lamellaires se trouvant dans la membrane séreuse et les tissus apparentés.

27. Procédé suivant la revendication 25, dans lequel lesdits corps lamellaires sont essentiellement identiques aux corps lamellaires se trouvant normalement dans un tissu enflammé ou dégénéré par ladite maladie.

28. Procédé suivant la revendication 25, dans lequel ladite maladie est le rhumatisme articulaire et lesdits corps lamellaires sont des corps lamellaires synoviaux.

29. Procédé suivant la revendication 25, 26, 27 ou 28, dans lequel lesdits constituants protéiniques sont choisis parmi l'ensemble constitué par l'apoprotéine SP-A, l'apoprotéine SP-B, l'apoprotéine SP-C et l'apoprotéine SP-D, et lesdits constituants phospholipidiques sont choisis parmi l'ensemble constitué par les phosphatidylcholine, phosphatidylinositol, phosphatidyléthanolamine, sphingomyéline, lécithine, phosphatidylglycérol, diphosphatidylglycérol, phosphatidylsérine et lysolécithine.

30. Procédé de diagnostic *in vitro* d'une maladie inflammatoire et dégénérative de tissus séreux et apparentés, ledit procédé comprenant l'incubation d'un antigène choisi parmi les corps lamellaires purifiés, les constituants protéiniques purifiés desdits corps lamellaires et les constituants phospholipidiques purifiés desdits corps lamellaires avec un échantillon de tissu séreux ou apparenté, de façon que les anticorps de l'échantillon soient liés à l'antigène, et la détection de la présence des anticorps liés.

31. Procédé suivant la revendication 30. dans lequel les constituants protéiniques sont choisis parmi l'apoprotéine SP-A, l'apoprotéine SP-B, l'apoprotéine SP-C et l'apoprotéine SP-D.

32. Procédé suivant la revendication 30 ou 31, dans lequel lesdits corps lamellaires sont des corps lamellaires synoviaux.

33. Dispositif pour utilisation dans le diagnostic d'une maladie inflammatoire et dégénérative de tissus séreux ou apparentés, ledit dispositif comprenant une plaque de microtitration ayant une paroi revêtue d'un antigène choisi parmi les corps lamellaires purifiés, leurs constituants protéiniques purifiés ou leurs constituants phospholipidiques purifiés.

34. Dispositif suivant la revendication 33, dans lequel les constituants protéiniques sont choisis parmi l'apoprotéine SP-A, l'apoprotéine SP-B, l'apoprotéine SP-C et l'apoprotéine SP-D.

35. Dispositif suivant la revendication 33, dans lequel lesdits corps lamellaires sont des corps lamellaires synoviaux.

## Patentansprüche

1. Pharmazeutische Zusammensetzung für die Behandlung einer Erkrankung aus einer Gruppe von entzündlichen und degenerativen Erkrankungen von serösem und verwandtem Gewebe, wobei die Zusammensetzung folgendes aufweist; (a) einen Immunkomplex aus einem Antigen, das aus der Gruppe ausgewählt ist, die aus Lamellenkörpern, Proteinbestandteilen dieser Lamellenkörper sowie Phospholipidbestandteilen dieser Lamellenkörper besteht, und einem für das genannte Antigen spezifischen Antikörper, und (b) einen pharmazeutisch akzeptablen Träger.

2. Zusammensetzung nach Anspruch 1, wobei das Antigen und der Antikörper in einem solchen Verhältnis anwesend sind, daß im wesentlichen sämtliche Bindungsstellen des Antigens durch den Antikörper blockiert sind, so daß bei Verabreichung an den Patienten das Antigen im wesentlichen keine Immunreaktion erzeugt.

3. Zusammensetzung nach Anspruch 1 oder 2, wobei die Lamellenkörper im wesentlichen identisch mit Lamellenkörpern sind, die normalerweise in einem durch die Erkrankung entzündeten oder degenerierten Gewebe gefunden werden.

4. Pharmazeutische Zusammensetzung für die Behandlung von entzündlichen und degenerativen Erkrankungen von serösem und verwandtem Gewebe, wobei die Zusammensetzung aufweist: gereinigte Lamellenkörper, Proteinbestandteile dieser Lamellenkörper oder Phospholipidbestandteile dieser Lamellenkörper sowie ein pharmazeutisch akzeptables Salz.

5. Pharmazeutische Zusammensetzung, die folgendes aufweist: T-Zellen oder T-Zellrezeptoren, die Lamellenkörper aus serösem und verwandtem Gewebe, Proteinbestandteile dieser Lamellenkörper oder Phospholipidbestandteiie dieser Lamellenkörper erkennen, und einen pharmazeutisch akzeptablen Träger.

6. Zusammensetzung nach Anspruch 1, 2 oder 5, wobei die Lamellenkörper Lamellenkörper sind, die in Serosa und verwandtem Gewebe gefunden werden.

7. Zusammensetzung nach Anspruch 1, 2, 5 oder 6, wobei die Lamellenkörper synoviale Lamellenkörper sind.

8. Zusammensetzung nach Anspruch 1, 2, 5 oder 6, wobei die Lamellenkörper im wesentlichen identisch mit Lamellenkörpern sind, die in einem Gewebe gefunden werden, das durch primärchronische Polyarthritis, rheumatisches Fieber, Lupus erythematodes, Arthritis psoriatica, Spondylarthropathia, Sjögrensches Syndrom, Reitersches Syndrom, Synovialitis, Osteoarthritis oder Tendosynovitis entzündet oder degeneriert ist.

9. Zusammensetzung nach einem der vorhergehenden Ansprüche, wobei die Proteinbestandteile aus der Gruppe ausgewählt sind, die aus Apoprotein SP-A, Apoprotein SP-B, Apoprotein SP-C und Apoprotein SP-D besteht, und die Phospholipidbestandteile aus der Gruppe ausgewählt sind, die aus Phosphatidylcholin, Phosphatidylinositol, Phosphatidylethanolamin, Sphingomyelin, Lecithin, Phosphatidylglycerin, Diphosphatidylglycerin, Phosphatidylserin und Lysolecithin besteht.

10. Verwendung - bei der Herstellung einer pharmazeutischen Zusammensetzung - eines Immunkomplexes, der folgendes aufweist: ein Antigen, das aus Lamellenkörpern, Proteinbestandteilen dieser Lamellenkörper oder Phospholipidbestandteilen dieser Lamellenkörper ausgewählt ist, und einen für dieses Antigen spezifischen Antikörper, zum Zweck der Behandlung von entzündlichen oder degenerativen Erkrankungen von serösem und verwandtem Gewebe bei einem Patienten.

11. Verwendung nach Anspruch 10, wobei das Antigen und der Antikörper in einem solchen Verhältnis anwesend sind, daß im wesentlichen sämtliche Bindungsstellen des Antigens von dem Antikörper blockiert sind, so daß bei Verabreichung an den Patienten das Antigen im wesentlichen keine Immunreaktion erzeugt.

12. Verwendung - bei der Herstellung einer pharmazeutischen Zusammensetzung - von β-adrenergischen Agonisten (Terbutalin), Phosphodiesterase-Hemmern (Isobutylmethylanthin), Choleratoxin, 8-bromocyclischem Adenosinmonophosphat, TPA, Calciumionophase, cholinergischen Agonisten oder Corticosteroiden zu dem Zweck, in einem Patienten die Freisetzung von oberflächenentspannendem Agens durch Lamellenkörper in serösem und verwandtem Gewebe, das durch Erkrankung entzündet oder degeneriert ist, zu stimulieren.

13. Verwendung - bei der Herstellung eines Impfstoffs - von T-Zellinien oder T-Zellrezeptoren, die Lamellenkörper von serösem und verwandtem Gewebe erkennen, von Proteinbestandteilen dieser Lamellenkörper oder von Phospholipidbestandteilen dieser Lamellenkörper zum Zweck des Impfens eines Patienten gegen entzündliche und degenerative Erkrankung von serösem und verwandtem Gewebe.

14. Verwendung - bei der Herstellung einer pharmazeutischen Zusammensetzung - von Lamellenkörpern, Proteinbestandteilen von Lamellenkörpern oder Phospholipidbestandteilen von Lamellenkörpern zum Zweck des Impfens eines Patienten, bei dem eine solche Erkrankung verhindert werden soll, zum Zweck der Verhinderung von entzündlichen und degenerativen Erkrankungen von serösem und verwandtem Gewebe, indem in einem Patienten eine Autoimmuntoleranz gegenüber Lamellenkörpern in diesem Gewebe, Proteinbestandteilen dieser Lamellenkörper oder Phospholipidbestandteilen dieser Lamellenkörper induziert wird.

15. Verwendung nach Anspruch 11 oder 14, wobei die Lamellenkörper solche Lamellenkörper sind, die in Serosa und verwandtem Gewebe gefunden werden.

16. Verwendung nach einem der Ansprüche 11 oder 14, wobei die Lamellenkörper im wesentlichen identisch mit Lamellenkörpern sind, die normalerweise in einem durch die Erkrankung entzündeten oder degenerierten Gewebe gefunden werden.

17. Verwendung nach einem der Ansprüche 11 bis 16, wobei die Erkrankung primärchronische Polyarthritis ist.

18. Verwendung nach einem der Ansprüche 11 bis 16, wobei die Erkrankung rheumatisches Fieber, Lupus erythematodes, Arthritis psoriatica, Spondylarthropathia, Sjögrensches Syndrom, Reitersches Syndrom, Synovialitis, Osteoarthritis oder Tendosynovitis ist.

19. Verwendung nach Anspruch 10, 11, 13 oder 14 oder nach einem der davon abhängigen Ansprüche 15 bis 18, wobei die Proteinbestandteile aus der Gruppe ausgewählt sind, die aus Apoprotein SP-A, Apoprotein SP-B, Apoprotein SP-C und Apoprotein SP-D besteht, und die Phospholipidbestandteile aus der Gruppe ausgewählt sind, die aus Phosphatidylcholin, Phosphatidylinositol, Phosphatidylethanolamin, Sphingomyelin, Lecithin, Phosphatidylglycerin, Diphosphatidylglycerin, Phosphatidylserin und Lysolecithin besteht.

20. Verfahren zum Herstellen einer pharmazeutischen Zusammensetzung zur Behandlung von entzündlichen und degenerativen Störungen von serösem und verwandtem Gewebe, wobei das Verfahren die folgenden Schritte aufweist: Identifizieren von stimulierenden Peptiden von Lamellenkörper-Apoproteinursprung, die bei Vereinigung mit komplexen Molekülen großer Histokompatibilität von T-Zellrezeptoren erkannt werden und eine spezifische Immunantwort auslösen, die einer solchen Erkrankung zugeordnet ist, und Herstellen einer Zusammensetzung von analogen Peptiden, die imstande sind, sich mit dem genannten Komplex in Konkurrenz mit den stimulierenden Peptiden zu vereinigen, jedoch von den T-Zellrezeptoren nicht erkannt werden können.

21. Verfahren nach Anspruch 20, wobei das Apoprotein von Lamellenkörpern ist, die in Serosa und verwandtem Gewebe gefunden werden.

22. Verfahren nach Anspruch 20, wobei das Apoprotein von Lamellenkörpern ist, die im wesentlichen identisch mit Lamellenkörpern sind, die normalerweise in einem Gewebe gefunden werden, das durch die genannte Erkrankung entzündet oder degeneriert ist.

23. Verfahren nach Anspruch 20, 21 oder 22, wobei die Erkrankung primärchronische Polyarthritis ist.

24. Verfahren nach Anspruch 20, 21 oder 22, wobei die Erkrankung rheumatisches Fieber, Lupus erythematodes, Arthritis psoriatica, Spondylarthropathia, Sjögrensches Syndrom, Reitersches Syndrom, Synovialitis, Osteoarthritis oder Tendosynovitis ist.

25. Verfahren zum Herstellen einer pharmazeutischen Zusammensetzung für die Behandlung von entzündlichen und degenerativen Erkrankungen von serösem und verwandtem Gewebe, wobei das Verfahren den folgenden Schritt aufweist: Vermischen eines Antigens, das aus der Gruppe ausgewählt ist, die aus Lamellenkörpern, Proteinbestandteilen dieser Lamellenkörper und Phospholipidbestandteilen dieser Lamellenkörper besteht, und eines dafür spezifischen Antikörpers in einem solchen Verhältnis, daß im wesentlichen sämtliche Bindungsstellen des Antigens von dem Antikörper blockiert sind, so daß bei Verabreichung an einen Patienten, der an einer solchen Erkrankung leidet, das Antigen im wesentlichen keine Immunreaktion erzeugt.

26. Verfahren nach Anspruch 25, wobei die Lamellenkörper solche Lamellenkörper sind, die in Serosa und verwandten Geweben gefunden werden.

27. Verfahren nach Anspruch 25, wobei die Lamellenkörper im wesentlichen identisch sind mit Lamellenkörpern, die normalerweise in einem Gewebe gefunden werden, das durch die Erkrankung entzündet oder degeneriert ist.

28. Verfahren nach Anspruch 25, wobei die Erkrankung primärchronische Polyarthritis ist und die Lamellenkörper synoviale Lamellenkörper sind,

29. Verfahren nach Anspruch 25, 26, 27 oder 28, wobei die Proteinbestandteile aus der Gruppe ausgewählt sind, die aus Apoprotein SP-A, Apoprotein SP-B, Apoprotein SP-C und Apoprotein SP-D besteht, oder die Phospholipidbestandteile aus der Gruppe ausgewählt sind, die aus Phosphatidylcholin, Phosphatidylinositol, Phosphatidylethanolamin, Sphingomyelin, Lecithin, Phosphatidylglycerin, Diphosphatidylglycerin, Phosphatidylserin und Lysolecithin besteht.

30. *In-vitro*-Verfahren zur Diagnostizierung einer entzündlichen und degenerativen Erkrankung von serösem und verwandtem Gewebe, wobei das Verfahren folgendes aufweist: Inkubieren eines Antigens, das aus gereinigten Lamellenkörpern, Proteinbestandteilen dieser Lamellenkörper und Phospholipidbestandteilen dieser Lamellenkörper ausgewählt ist, mit einer serösen oder verwandten Gewebsprobe, so daß Antikörper in der Probe an das Antigen gebunden werden, und Nachweisen der Anwesenheit der gebundenen Antikörper.

31. Verfahren nach Anspruch 30, wobei die Proteinbestandteile aus Apoprotein SP-A, Apoprotein SP-B, Apoprotein SP-C und Apoprotein SP-D ausgewählt sind.

32. Verfahren nach Anspruch 30 oder 31, wobei die Lamellenkörper synoviale Lamellenkörper sind.

33. Vorrichtung zur Verwendung bei der Diagnose einer entzündlichen und degenerativen Erkrankung von serösem oder verwandtem Gewebe, wobei die vorrichtung eine Mikrotiterplatte aufweist, die eine Vertiefung besitzt, die mit einem Antigen überzogen ist, das aus gereinigten Lamellenkörpern, Proteinbestandteilen davon oder Phospholipidbestandteilen davon ausgewählt ist.

34. Vorrichtung nach Anspruch 33, wobei die Proteinbestandteile aus Apoprotein SP-A, Apoprotein SP-B, Apoprotein SP-C und Apoprotein SP-D ausgewählt sind.

35. Vorrichtung nach Anspruch 33, wobei die Lamellenkörper synoviale Lamellenkörper sind.
